(19) **Europäisches Patentamt European Patent Office Office européen des brevets**

(11) **EP 1 949 913 B1**

(12) **EUROPEAN PATENT SPECIFICATION**

(45) Date of publication and mention of the grant of the patent:
**02.06.2010 Bulletin 2010/22**

(51) Int Cl.:
*A61K 39/39* (2006.01)   *A61K 39/395* (2006.01)
*C07H 21/02* (2006.01)   *C07K 14/52* (2006.01)

(21) Application number: **06830863.4**

(22) Date of filing: **05.10.2006**

(86) International application number:
**PCT/ES2006/000554**

(87) International publication number:
**WO 2007/042583 (19.04.2007 Gazette 2007/16)**

(54) **IMMUNOSTIMULATORY COMBINATION FOR THE PROPHYLACTICS AND TREATMENT OF HEPATITIS C**

IMMUNSTIMULATORISCHE KOMBINATION ZUR VORBEUGUNG UND BEHANDLUNG VON HEPATITIS C

COMBINAISON IMMUNOSTIMULATRICE POUR LA PROPHYLAXIE ET LE TRAITEMENT DE L'HÉPATITE C

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **07.10.2005 ES 200502446**
**09.06.2006 ES 200601563**

(43) Date of publication of application:
**30.07.2008 Bulletin 2008/31**

(73) Proprietor: **Proyecto de Biomedicina Cima, S.L.**
**31180 Zizur Mayor - Navarra (ES)**

(72) Inventors:
• **ZABALETA AZPIROZ, Aintzane**
**E-31008 Pamplona (Navarra) (ES)**
• **BORRAS CUESTA, Francisco**
**E-31008 Pamplona (Navarra) (ES)**
• **PRIETO VALTUEÑA, Jesús**
**E-31008 Pamplona (Navarra) (ES)**
• **SAROBE UGARRIZA, Pablo**
**E-31008 Pamplona (Navarra) (ES)**
• **LASARTE SAGASTIBELZA, Juan José**
**E-31008 Pamplona (Navarra) (ES)**

(74) Representative: **Ungria Lopez, Javier**
**Avda. Ramón y Cajal, 78**
**28043 Madrid (ES)**

(56) References cited:
**WO-A-2005/012509   WO-A2-02/14362**

• **LAURA ARRIBILLAGA ET AL.: "Vaccination with an adenoviral vector encoding hepatitis C virus (HCV) NS3 protein protects against infection with HCV-recombinant vaccinia virus" VACCINE, vol. 21, 2002, pages 202-210, XP002511030**
• **ROUAS R. ET AL.: 'Poly(I:C) used for human dendritic cell maturation preserves their ability to secondarily secrete bioactive IL-12' INTERNATIONAL IMMUNOLOGY vol. 16, no. 5, May 2004, pages 767 - 773, XP003012396**
• **AHONEN C.L. ET AL.: 'Combined TLR and CD40 triggering induces potent CD8+ T cell expansion with variable dependence on type I IFN' JOURNAL OF EXPERIMENTAL MEDICINE vol. 199, no. 6, March 2004, pages 775 - 784, XP003012397**

EP 1 949 913 B1

SEQUENCE LISTING

<110> PROYECTO DE BIOMEDICINA CIMA S.L.

<120> IMMUNO-STIMULANT COMBINATION FOR PROPHYLAXIS AND TREATMENT OF HEPATITIS C

<130> 05009

<160> 7

<170> Patentin Version 3.1

<210> SEQ. ID. NO.: 1
<211> 631
<212> PRT
<213> Virus hepatitis C

<220>
<221> MISC_FEATURE
<223> Non-structural NS3 protein

<400> 1

```
Ala Pro Ile Thr Ala Tyr Ala Gln Gln Thr Arg Gly Leu Leu Gly Cys
1               5                  10                  15

Ile Ile Thr Ser Leu Thr Gly Arg Asp Lys Asn Gln Val Glu Gly Glu
            20                  25                  30

Val Gln Ile Val Ser Thr Ala Ala Gln Thr Phe Leu Ala Thr Cys Ile
        35                  40                  45

Asn Gly Val Cys Trp Thr Val Tyr His Gly Ala Gly Thr Lys Thr Ile
    50                  55                  60
```

Ala Ser Ser Lys Gly Pro Val Ile Gln Met Tyr Thr Asn Val Asp Gln
65          70          75             80

Asp Leu Val Gly Trp Pro Ala Pro Gln Gly Ala Arg Ser Leu Thr Pro
            85          90             95

Cys Thr Cys Gly Ser Ser Asp Leu Tyr Leu Val Thr Arg His Ala Asp
            100          105             110

Val Ile Pro Val Arg Arg Arg Gly Asp Ser Arg Gly Ser Leu Leu Ser
        115          120             125

Pro Arg Pro Ile Ser Tyr Leu Lys Gly Ser Ser Gly Gly Pro Leu Leu
    130          135             140

Cys Pro Ala Val His Ala Val Gly Ile Phe Arg Ala Ala Val Cys Thr
145          150          155             160

Arg Gly Val Ala Lys Ala Val Asp Phe Ile Pro Val Glu Gly Leu Glu
            165          170             175

Thr Thr Met Arg Ser Pro Val Phe Ser Asp Asn Ser Ser Pro Pro Ala
            180          185             190

Val Pro Gln Ser Tyr Gln Val Ala His Leu His Ala Pro Thr Gly Ser
    195          200          205

Gly Lys Ser Thr Lys Val Pro Ala Ala Tyr Ala Ala Gln Gly Tyr Lys
    210          215          220

Val Leu Val Leu Asn Pro Ser Val Ala Ala Thr Leu Gly Phe Gly Ala
225          230          235             240

Tyr Met Ser Lys Ala His Gly Ile Asp Pro Ile Ile Arg Thr Gly Val
            245          250          255

Arg Thr Ile Thr Thr Gly Ser Pro Ile Thr Tyr Ser Thr Tyr Gly Lys
        260          265          270

Phe Leu Ala Asp Gly Gly Cys Ser Gly Gly Ala Tyr Asp Ile Ile Ile
        275          280          285

Cys Asp Glu Cys His Ser Thr Asp Ala Thr Ser Ile Leu Gly Ile Asp
    290          295          300

```
His Phe Leu Ser Gln Thr Lys Gln Ser Gly Glu Asn Leu Pro Tyr Leu
545             550             555                         560


Val Ala Tyr Gln Ala Thr Val Cys Ala Arg Ala Gln Ala Pro Pro Pro
                565             570                 575


Ser Trp Asp Gln Met Trp Lys Cys Leu Ile Arg Leu Lys Pro Thr Leu
                580             585             590


His Gly Pro Thr Pro Leu Leu Tyr Arg Leu Gly Ala Val Gln Asn Glu
            595             600                 605


Ile Thr Leu Thr His Pro Ile Thr Lys Tyr Ile Met Thr Cys Met Ser
    610             615                 620


Ala Asp Leu Glu Val Val Thr
625             630
```

<210> SEQ. ID. NO.: 2
<211> 635
<212> PRT
<213> Hepatitis C virus

<220>
<221> MISC_PEATURE
<223> Non-structural NS3 protein
<400> 2

```
Ala Pro Ile Thr Ala Tyr Ser Gln Gln Thr Arg Gly Leu Leu Gly Cys
1               5               10              15


Ile Ile Thr Ser Leu Thr Gly Arg Asp Lys Asn Gln Val Asp Gly Glu
            20              25              30


Val Gln Val Leu Ser Thr Ala Thr Gln Ser Phe Leu Ala Thr Cys Val
        35              40              45


Asn Gly Val Cys Trp Thr Val Tyr His Gly Ala Gly Ser Lys Thr Leu
        50              55              60
```

Thr Val Leu Asp Gln Ala Glu Thr Ala Gly Ala Arg Leu Thr Val Leu
305          310          315               320

Ala Thr Ala Thr Pro Pro Gly Ser Val Thr Val Pro His Pro Asn Ile
            325          330               335

Glu Glu Val Ala Leu Ser Thr Thr Gly Glu Ile Pro Phe Tyr Gly Lys
            340          345               350

Ala Ile Pro Leu Glu Ala Ile Lys Gly Gly Arg His Leu Ile Phe Cys
            355          360               365

His Ser Lys Lys Lys Cys Asp Glu Leu Ala Ala Lys Leu Val Ala Leu
    370              375          380

Gly Val Asn Ala Val Ala Tyr Tyr Arg Gly Leu Asp Val Ser Val Ile
385              390          395               400

Pro Ala Ser Gly Asp Val Val Val Val Ala Thr Asp Ala Leu Met Thr
            405          410               415

Gly Phe Thr Gly Asp Phe Asp Ser Val Ile Asp Cys Asn Thr Cys Val
            420          425               430

Thr Gln Thr Val Asp Phe Ser Leu Asp Pro Thr Phe Thr Ile Glu Thr
    435              440              445

Thr Thr Leu Pro Gln Asp Ala Val Ser Arg Thr Gln Arg Arg Gly Arg
    450              455          460

Thr Gly Arg Gly Lys Pro Gly Ile Tyr Arg Phe Val Thr Pro Gly Glu
465              470          475               480

Arg Pro Ser Gly Met Phe Asp Ser Ser Val Leu Cys Glu Cys Tyr Asp
            485          490               495

Ala Gly Cys Ala Trp Tyr Glu Leu Thr Pro Ala Glu Thr Thr Val Arg
        500          505          510

Leu Arg Ala Tyr Met Asn Thr Pro Gly Leu Pro Val Cys Gln Asp His
    515              520          525

Leu Glu Phe Trp Glu Gly Val Phe Thr Gly Leu Thr His Ile Asp Ala
    530              535          540

13

```
Ala Gly Pro Lys Gly Pro Ile Thr Gln Met Tyr Thr Asn Val Asp Gln
65              70              75                          80

Asp Leu Val Gly Trp Pro Ala Pro Pro Gly Ala Arg Ser Met Thr Pro
                85              90                          95

Cys Thr Cys Gly Ser Ser Asp Leu Tyr Leu Val Thr Arg His Ala Asp
            100             105 ·             110

Val Val Pro Val Arg Arg Arg Gly Asp Ser Arg Gly Ser Leu Leu Ser
        115             120                 125

Pro Arg Pro Ile Ser Tyr Leu Lys Gly Ser Ser Gly Gly Pro Leu Leu
    130             135  ·             140

Cys Pro Ser Gly His Val Val Gly Ile Phe Arg Ala Ala Val Cys Thr
145             150             155                 160

Arg Gly Val Ala Lys Ala Val Asp Phe Ile Pro Val Glu Ser Met Glu
            165             170                 175

Thr Thr Met Arg Ser Pro Val Phe Thr Asp Asn Ser Ser Pro Pro Ala
            180             185                 190

Val Pro Gln Thr Phe Gln Val Ala His Leu His Ala Pro Thr Gly Ser
    195             200                 205

Gly Lys Ser Thr Lys Val Pro Ala Ala Tyr Ala Ala Gln Gly Tyr Lys
    210             215                 220

Val Leu Val Leu Asn Pro Ser Val Ala Ala Thr Leu Gly Phe Gly Ala
225             230                 235                 240

Tyr Met Ser Lys Ala His Gly Ile Glu Pro Asn Ile Arg Thr Gly Val
            245             250                 255

Arg Thr Ile Thr Thr Gly Gly Pro Ile Thr Tyr Ser Thr Tyr Cys Lys
            260             265                 270

Phe Leu Ala Asp Gly Gly Cys Ser Gly Gly Ala Tyr Asp Ile Ile Ile
    275             280                 285

Cys Asp Glu Cys His Ser Thr Asp Ser Thr Thr Ile Leu Gly Ile Gly
    290             295                 300
```

EP 1 949 913 B1

```
Thr Val Leu Asp Gln Ala Glu Thr Ala Gly Ala Arg Leu Val Val Leu
305             310             315             320

Ala Thr Ala Thr Pro Pro Gly Ser Ile Thr Val Pro His Pro Asn Ile
            325             330             335

Glu Glu Val Ala Leu Ser Asn Thr Gly Glu Ile Pro Phe Tyr Gly Lys
            340             345             350

Ala Ile Pro Ile Glu Ala Ile Lys Gly Gly Arg His Leu Ile Phe Cys
            355             360             365

His Ser Lys Lys Lys Cys Asp Glu Leu Ala Ala Lys Leu Thr Gly Leu
    370             375             380

Gly Leu Asn Ala Val Ala Tyr Tyr Arg Gly Leu Asp Val Ser Val Ile
385             390             395             400

Pro Thr Ser Gly Asp Val Val Val Ala Thr Asp Ala Leu Met Thr
            405             410             415

Gly Phe Thr Gly Asp Phe Asp Ser Val Ile Asp Cys Asn Thr Cys Val
            420             425             430

Thr Gln Thr Val Asp Phe Ser Leu Asp Pro Thr Phe Thr Ile Glu Thr
        435             440             445

Thr Thr Leu Pro Gln Asp Ala Val Ser Arg Ala Gln Arg Arg Gly Arg
    450             455             460

Thr Gly Arg Gly Arg Ser Gly Ile Tyr Arg Phe Val Thr Pro Gly Glu
465             470             475             480

Arg Pro Ser Gly Met Phe Asp Ser Ser Val Leu Cys Glu Cys Tyr Asp
            485             490             495

Ala Gly Cys Ala Trp Tyr Glu Leu Thr Pro Ala Glu Thr Ser Val Arg
        500             505             510

Leu Arg Ala Tyr Leu Asn Thr Pro Gly Leu Pro Val Cys Gln Asp His
    515             520             525

Leu Glu Phe Trp Glu Ser Val Phe Thr Gly Leu Thr His Ile Asp Ala
    530             535             540
```

15

```
His Phe Leu Ser Gln Thr Lys Gln Ala Gly Asp Asn Leu Pro Tyr Leu
545                 550                 555                 560


Val Ala Tyr Gln Ala Thr Val Cys Ala Arg Ala Gln Ala Pro Pro Pro
                565                 570                 575


Ser Trp Asp Gln Met Trp Lys Cys Leu Ile Arg Leu Lys Pro Thr Leu
                580                 585                 590


His Gly Pro Thr Pro Leu Leu Tyr Arg Leu Gly Ala Val Gln Asn Glu
                595                 600                 605


Val Thr Leu Thr His Pro Ile Thr Lys Tyr Ile Met Ala Cys Met Ser
            610                 615                 620


Ala Asp Leu Glu Val Val Thr Ser Thr Trp Val
625                 630                 635
```

<220>
<223> Chimeric recombinant protein obtained from the proteins NS2 and NS3 of the hepatitis C virus
<400> 3

```
Gly Arg Glu Ile Leu Leu Gly Pro Ala Asp Gly Met Ala Ser Lys Gly
1               5                   10                  15


Trp Arg Leu Leu Ala Pro Ile Thr Ala Tyr Ala Gln Gln Thr Arg Gly
            20                  25                  30


Leu Leu Gly Cys Ile Ile Thr Ser Leu Thr Gly Arg Asp Lys Asn Gln
            35                  40                  45


Val Glu Gly Glu Val Gln Ile Val Pro Thr Ala Ala Gln Thr Phe Leu
        50                  55                  60


Ala Thr Cys Ile Asn Gly Val Cys Trp Thr Val Tyr His Gly Ala Gly
65                  70                  75                  80
```

<210> SEQ. ID. NO.: 3
<211> 528
<212> PRT
<213> Artificial Sequence

```
Thr Arg Thr Ile Ala Ser Pro Lys Gly Pro Val Ile Gln Met Tyr Ser
             85              90                      95

Asn Val Asp Lys Asp Leu Val Gly Trp Pro Ala Pro Gln Gly Ser Arg
            100             105          .      110

Ser Leu Ala Pro Cys Thr Cys Gly Ser Ser Asp Leu Tyr Leu Val Thr
            115             120             125

Lys His Ala Asp Val Ile Pro Val Arg Arg Arg Gly Asp Ser Arg Gly
    .       130             135             140

Ser Leu Leu Ser Pro Arg Pro Ile Ser Tyr Leu Lys Gly Ser Ser Gly
145             150             155             160

Gly Pro Leu Leu Cys Pro Val Gly His Ala Val Gly Ile Phe Arg Ala
            165             170             175

Ala Val Cys Thr Arg Gly Val Ala Lys Ala Ala Asp Phe Ile Pro Val
            180             185             190

Glu Asn Leu Glu Thr Thr Met Arg Ser Pro Val Phe Thr Asp Asn Ser
            195             200             205

Ser Pro Pro Val Val Pro Gln Ser Phe Gln Val Ala His Leu His Ala
210             215             220

Pro Thr Gly Ser Gly Lys Ser Thr Lys Val Pro Ala Ala Tyr Ala Ala
225             230             235             240

Gln Gly Tyr Lys Val Leu Val Leu Asn Pro Ser Val Ala Ala Thr Leu
            245             250             255

Gly Phe Gly Ala Tyr Met Ser Lys Ala His Gly Ile Asp Pro Asn Ile
            260             265             270

Arg Thr Gly Val Arg Thr Ile Thr Thr Gly Ser Pro Ile Thr Tyr Ser
            275             280             285

Thr Tyr Gly Lys Phe Leu Ala Asp Gly Gly Cys Ala Gly Gly Ala Tyr
    290             295     .       300

Asp Ile Ile Ile Cys Asp Glu Cys His Ser Thr Asp Ala Thr Ser Ile
305             310             315             320
```

```
Leu Gly Ile Gly Thr Val Leu Asp Gln Gly Glu Thr Ala Gly Ala Lys
                325                 330                 335

Leu Val Val Phe Ala Thr Ala Thr Pro Pro Gly Ser Val Thr Val Pro
                340                 345                 350

His Pro Asn Ile Glu Glu Val Ala Leu Ser Thr Thr Gly Glu Ile Pro
                355                 360                 365

Phe Tyr Gly Lys Ala Ile Pro Leu Glu Val Ile Lys Gly Gly Arg His
        370                 375                 380

Leu Ile Phe Cys His Ser Lys Arg Lys Cys Asp Glu Leu Ala Thr Lys
385                 390                 395                 400

Leu Val Ala Met Gly Ile Asn Ala Val Ala Tyr Tyr Arg Gly Leu Asp
                405                 410                 415

Val Ser Val Ile Pro Thr Ser Gly Asp Val Val Val Val Ala Thr Asp
                420                 425                 430

Ala Leu Met Thr Gly Tyr Thr Gly Asp Phe Asp Ser Val Ile Asp Cys
            435                 440                 445

Asn Thr Cys Val Thr Gln Thr Val Asp Phe Ser Leu Asp Pro Thr Phe
        450                 455                 460

Thr Ile Glu Thr Thr Thr Leu Pro Gln Asp Ala Val Ser Arg Thr Gln
465                 470                 475                 480

Arg Arg Gly Arg Thr Gly Arg Gly Lys Pro Gly Ile Tyr Arg Phe Val
                485                 490                 495

Ala Pro Gly Glu Arg Pro Ser Gly Met Phe Asp Ser Ser Val Leu Cys
                500                 505                 510

Glu Cys Tyr Asp Ala Gly Cys Ala Trp Tyr Glu Leu Thr Pro Ala Glu
            515                 520                 525
```

<210> SEQ. ID. NO.: 4
<211> 10
<212> PRT
<213> Hepatitis C virus

<220>
<221> misc_feature
<223> Epitope 1038-1047 corresponding to the NS3 viral protein

<400> 4

```
                    Gly Leu Leu Gly Cys Ile Ile Thr Ser Leu
                    1               5                   10
```

<210> SEQ. ID. NO.: 5
<211> 9
<212> PRT
<213> Hepatitis C virus

<220>
<221> misc_feature
<223> Epitope 1073-1081 corresponding to the NS3 viral protein

<400> 5

```
                    Cys Val Asn Gly Val Cys Trp Thr Val
                    1               5
```

<210> SEQ. ID. NO.: 6
<211> 10
<212> PRT
<213> Hepatitis C virus

<220>
<221> misc_feature
<223> Epitope 1406-1415 corresponding to the viral protein

<400> 6

```
                    Lys Leu Val Ala Leu Gly Ile Asn Ala Val
                    1               5                   10
```

<210> SEQ. ID. NO.: 7
<211> 9
<212> PRT
<213> Hepatitis C virus

<400> 7

```
                    Gly Ala Val Gln Asn Glu Val Thr Leu
                    1               5
```

**Description**

TECHNICAL FIELD OF THE INVENTION

The present invention relates to an immuno-stimulant combination for prophylaxis and treatment of hepatitis C, which incorporates the NS3 protein of HCV, together with adjuvants selected for their capacity to induce specific potent and lasting CD8+ and CD4+ responses against the HCV virus.

STATE OF THE ART

With an estimated world prevalence of over 170 million people infected, infection by the hepatitis C virus (HCV) today implies a heavy burden for public health. And this is a prevalence that will presumably remain invariable in the coming years.

Infection by HCV is characterised by a high tendency towards chronicity. HCV persists in 70% of infected individuals, 20% of whom develop cirrhosis and 2.5% evolve to producing cancer of the liver.

The current reference therapeutic tool is therapeutic protocols based on the use of interferon. Nevertheless, these antiviral therapies are economically costly, relatively toxic and only effective in 50-60% of patients treated. It is therefore necessary and desirable to develop new therapeutic strategies that are more effective and better tolerated by patients.

An updated review of HCV can be found in Nature ("Insights: Hepatitis C". Nature 2005, Supplements; Vol. 436, Nr. 7053, pp 929-978).

Although, regrettably, we do not yet have an effective vaccine against hepatitis C virus, there is experimental data and evidence that leads one to think that an effective vaccine is possible. Although antiviral antibodies are synthesised in response to the infection, the chronic state is characterised by the absence of cellular immune responses on the part of cytotoxic T-cells (CD8+) and helper T-cells (CD4+). So, it is postulated that the HCV has developed strategies permitting it to specifically evade the antiviral immune responses, where the power and quality of the cytotoxic T and helper T responses determine whether the patients will recover (either spontaneously or in response to a treatment) or whether they will develop a chronic infection.

The main objective of any vaccine is to stimulate the antigen specific acquired immunity, the mediators of which are the B and T-Lymphocytes. In this context, the antigen presenting cells (APCs) play an important role in the initiation of the specific immune responses and in particular in the activation of T-Lymphocytes. APCs, mainly dendritic cells, capture antigens at the peripheral organs and, after receiving an activation stimulus, they migrate to the lymphatic organs. There, the dendritic cells do present at their surface, joined to actual molecules of the major histocompatibility complex MHC, the peptide products derived from the degradation of the antigens (epitopes), and they simultaneously produce chymokines and cytokines in order to attract and activate T-cells. The activation process of dendritic cells, also known as maturation, is characterised by a high expression of MHC molecules (signal 1), co-stimulator molecules (signal 2) and polariser cytokines such as interleukin-12 (IL-12) (signal 3). The maturation is induced by factors such as pathogen components or molecules of the host that are frequent in inflammation or cell damage processes. These factors act on the dendritic cells via specific receptors for products derived from microorganisms, such as TLR type receptors (Toll-like receptors), receptors for cytokines (TNF-$\alpha$, IL-1, IFN-$\alpha$) or receptors for ligands on the cell surfaces (e.g., CD40).

Stimulation and activation of the different populations of T-cells by the APCs is restricted by the type of MHC molecules on the one hand, and, on the other, by the characteristics of the epitopes which form complexes with those MHC molecules. So, for example, certain fragments has been identified of viral proteins which specifically induce the activation of cytotoxic CD8+ T-Lymphocytes (CTL), known as lymphocyte epitopes or CD8+ T-cells or CD8+ epitopes; or epitopes which specifically induce the activation of CD4+ helper T-Lymphocytes (HTL), CD4+ epitopes. The database "HCV Immunology Database" (http://hcv.lanl.gov/content/immuno/immuno-main.html) compiles the epitopes for T-Lymphocytes, both of CD8+ CTL and of CD4+ HTL, identified on the basis of viral proteins of different strains and isolates of the hepatitis C virus.

The development of immunisation protocols based on the use of epitopes in the form of peptides thus requires the previous selection of those peptides that are suitable for each individual, depending on the MHC molecules they present. This implies that, depending on the MHC of each individual, a particular combination of peptides would have to be chosen which would be able to behave as epitopes in that context. The use of large antigens permits this problem to be overcome, since they are normally polyepitopic and within their sequence they present various epitopes, both for CD8+ CTL and for CD4+ HTL, which can be presented by MHC molecules of different individuals. In this way, a single antigen can be used as a vaccine in individuals with different MHC.

Within the different proteins of HCV, core and NS3 present great immunogenicity and in those individuals which get over the infection, potent CD8+ CTL and CD4+ HTL responses are detected against them. Nevertheless, there exist data which show that core can also have deleterious effects for the cells of the immune system, when it is in contact with them, which makes it inadvisable as an antigen in vaccination strategies. On the other hand, NS3 is a protein that has scarcely demonstrated this type of effect and could be a good candidate as an antigen for induction of CD8+ CTL and CD4+ HTL responses.

In this sense, WO 2002/014362 A2 discloses the use of HCV NS3 protein as antigen in the manufacture of vaccines for prophylaxis and treatment of HCV infection. NS3 may be accompanied by adjuvant ribavirin, which may be found in the same pharmaceutical composition or in a different one. Additionally, adjuvant administration may be simultaneous to that of the antigen or at a different moment. This document, however, does not demonstrate that ribavirin enhances CD8 immune response against NS3.

The CD4+ HTL play a role in acquired immunity, among other mechanisms by means of APC activation, CTL activation and memory induction. In particular, it has been described that the CD4+ cells specific for HCV are necessary for maintenance of antiviral CTL (Grakoui A. et al., "HCV persistence and immune evasion in the absence of memory T-cell help"; Science, 2003; 302: 659-662). Therefore, an effective vaccine against the hepatitis C virus has to provide the maximum power in the induction of not just CD8+ CTL responses but also of CD4+ HTL responses. Such a vaccine will therefore require a selection of specific antigens that will provide those responses.

Nevertheless, it does not seem that a combination of antigens can, on its own, be capable of providing an effective vaccine against HCV. Given that the maturation of dendritic cells is a requirement for the effective initiation and activation of T-Lymphocytes, such a vaccine could benefit from the inclusion into the immuno-stimulant combination of some adjuvants, which would stimulate the maturation of the dendritic cells. As adjuvants, use could be made of ligands of TLR receptors, of cytokine receptors or of receptors for intercellular ligands already cited, or better yet a synergic combination of those adjuvants.

So, for example, Rouas et al. (International Immunology, May 2004, 16(5): 767-773) discloses the use *in vitro* of CD40L, IFN-γ and poli(I:C), a synthetic ligand of TLR3, in a pharmaceutical composition producing mature dendritic cells. It shows that dendritic cells matured with poli(I:C) are the only ones that secrete IL-12p70 after stimulation with CD40L, which does not occur upon maturation with IFN-γ. It also discloses dendritic cell maturation when they have been incubated with both CD40L and poly(I:C). Mature dendritic cells are very important in the immune response as the IL-12 that they secrete activates both Th1 lymphocytes and cytotoxic T lymphocytes.

Further, US2004/0141950 describes immuno-stimulant combinations which include an antagonist of TLRs and an antagonist of molecules of the superfamilies of the tumour necrosis factor (TNF) or of its receptors (TNFR), which can also include an antigen. Among the numerous possible combinations it presents the combination of a ligand of CD40 (an anti-CD40 antibody) and of poly(I:C), a combination for which a synergic effect is demonstrated in the expansion of CD8+ T-Lymphocytes. Likewise, Ahonen et al. (J. Exp. Med. 2004; 199: 775-784) present data on the synergic capacity of TLR/CD40 agonists for inducing the expansion and differentiation of antigen specific CD8+ CTL in a manner that is independent of CD4+ T-Lymphocytes. Although these works describe the capacity of the TLR/CD40 for activating CD8+ T-Lymphocytes of antigen specific memory, said works do not permit it to be established whether the combination of TLR/CD40 agonists can also boost the CD4+ HTL responses.

In the case of infection by HCV, clear differences have been found in the CD4+ HTL responses when infected patients are compared to patients who have been able to eliminate the infection. Nevertheless, although with lesser intensity than in cured patients, CD8+ CTL responses are still detectable in infected patients. Therefore, although the CTL behave as an important effector population in clearing up HCV infection, the CD4+ cells also play an important role in controlling the disease. Moreover, it has been described that the induction of CD4+ T-Lymphocytes is important for maintenance of the antiviral CTL responses (Grakoui A. et al., "HCV persistence and immune evasion in the absence of memory T-cell help"; Science, 2003; 302: 659-662). These data suggest that for the vaccination and therapy of viral diseases due to HCV, the induction of potent and lasting antiviral responses, both CD8+ and CD4+, are important.

It is therefore the object of the present invention to select immuno-stimulant combinations of antigens and adjuvants suitable for the prophylaxis and treatment of hepatitis C, which will provide a stimulation of both CD8+ and CD4+ responses that are more potent, complete and lasting.

DETAILED DESCRIPTION OF THE INVENTION

A first object of the invention relates to an immuno-stimulant combination for prophylaxis and treatment of hepatitis C, hereinafter referred as the inventive immuno-stimulant combination, which comprises a TLR3 agonist, a CD40 agonist or a sequence of DNA that codes it, and a polypeptide which comprises the NS3 protein of the hepatitis C virus, or a fragment of said NS3 protein with capacity for inducing CD8+ and CD4+ responses.

A "TLR3 agonist" refers to a ligand which can be combined or joined to the TLR3 receptors ("toll like receptor 3") and produce a cellular response. TLR3 is a receptor for double stranded RNA which transmits signals that activate NF-κB and the production interferons (IFN) of type I (IFN-α and IFN-β) and which stimulate the maturation of the dendritic cells. Mice lacking TLR3 expression showed a reduction in their responses to poly(I:C) - a TLR3 ligand similar to double stranded RNA generated during the replication of virus of the HCV type -, along with resistance to the lethal effect of poly(I:C) when sensitised with D-galactosamine and a reduction in the production of inflammatory cytokines (Alexopoulou et al. Nature, 2001, Vol. 413, pp. 732-738). In a particular embodiment of the invention, said ligand of TLR3 can be a viral double stranded RNA or a double chain of polyinosinic-polycytidylic acid, poly(I:C).

A "CD40 agonist" refers to a ligand, which can be combined or joined to the CD40 receptors likewise inducing a cellular response. CD40 is a molecule expressed in the membrane of different cell types, such as B-Lymphocytes or antigen presenting cells (macrophages, dendritic cells, etc.). The natural ligand of CD40 (CD40L or CD154) is mainly expressed in T-Lymphocytes which have been activated following recognition of the antigen. The interaction of CD40L with CD40 present in the antigen presenter cell induces the maturation of the latter. This phenomenon, in a way similar to the stimuli coming from pathogens, causes the antigen-presenting cell to have a greater capacity for inducing immunitary responses. So, the CD40 agonist of the inventive immuno-stimulant composition refers on the one hand to the CD40L ligand or to a fragment of that CD40L which conserves the capacity for joining to CD40 and inducing a cellular or immune response. In a particular embodiment, the ligand can be a specific antibody to CD40 (anti-CD40) or a fragment thereof which conserves the capacity for joining to CD40. Moreover, the CD40 ligand or its fragment can be present in the immuno-stimulant combination either in the form of protein or also as a recombinant nucleic acid (DNA) which codes that ligand, for example in a viral vector for transference or gene therapy.

An "antigen" refers to any substance which is capable of inducing an immune response, both humoral and cellular, in the organism of an individual (man or an animal), or which can induce a cellular immune response (expansion, activation and/or maturation of immune cells, production of cytokines, or antibodies) when it comes into contact with immunitary cells. In particular, an antigen can be a viral protein, a peptide or a fragment of said viral protein, a recombinant protein of such viral proteins or even a synthetic peptide capable of inducing the signalled responses.

A "CD8+ inducer epitope" refers to a fragment or partial polypeptide chain of an antigen that is capable of specifically inducing the activation of CD8+ cytotoxic T-Lymphocytes (CTL). A "CD4+ inducer epitope" refers to a fragment of partial polypeptide chain of an antigen that is capable of specifically inducing the activation of CD4+ helper T-Lymphocytes (HTL).

"NS3 protein" refers to the non-structural protein NS3 of the hepatitis C virus, a protein of 67 kDa which includes 2 domains, a serin-proteinase covering 189 amino acids of the N-terminal end and a domain with helicase-nucleoside triphosphatase activity covering 442 amino acids of the C-terminal end. The sequence of the NS3 protein included in the polypeptide of the inventive immuno-stimulant combination can correspond to any strain or isolate of the hepatitis C virus, in particular any strain or isolate of the human hepatitis C virus. In a particular embodiment, the polypeptide, which comprises the NS3 protein, has been obtained by recombinant technology. In a specific non-limiting embodiment of the invention, a recombinant NS3 protein is used with a sequence SEQ ID. NO: 1 (corresponding to Genebank Accession numbers DQ068198.1 and AAY84763.1, VRL 28-NOV-2005). We have also used another recombinant protein sequence SEQ ID. NO: 2 (corresponding to Genebank Accession number D90208).

In another alternative embodiment of the invention, it is possible to also use a polypeptide, which comprises a fragment of the protein NS3, in such a way that said fragment is capable of inducing CD4+ and CD8+ responses. Therefore, said fragment will have to include at least one CD8+ inducer epitope and one CD4+ inducer epitope.

In a specific embodiment, the inventive immuno-stimulant combination comprises poly(I:C), an anti-CD40 antibody, and a polypeptide containing the NS3 protein.

In a preferred embodiment of the invention, the immuno-stimulant combination possesses all the components forming part of the same pharmaceutical composition, where each one of the components is present in pharmaceutically acceptable quantities. Furthermore, the invention also refers to said pharmaceutical composition.

In another specific embodiment of the present invention, the components of the immuno-stimulant combination are to be found forming part of at least two pharmaceutical compositions. Likewise, the invention refers to the use of said immuno-stimulant combination **characterised in that** said pharmaceutical compositions are administered simultaneously. In another embodiment of the invention, the use of said immuno-stimulant combination is **characterised in that** said pharmaceutical compositions are administered at different moments, via the same administration route or via different routes. So, one specific embodiment of the invention refers to a kit for the administration of the immuno-stimulant combination described above, **characterised in that** it comprises at least two different pharmaceutical compositions.

In another aspect, the invention refers to a method for producing an immune response to the hepatitis C virus **characterised in that** it consists of administering a stimulating combination defined above, in an effective quantity for inducing an immune response. In a preferred embodiment, the method of the invention consists of a prophylactic treatment. In a more preferred embodiment, the method of the invention consists of a therapeutic treatment.

Finally, the invention also refers to a vaccine against hepatitis C virus, **characterised in that** it comprises an immuno-stimulant combination defined above and forming the object of this invention.

BRIEF DESCRIPTION OF THE FIGURES

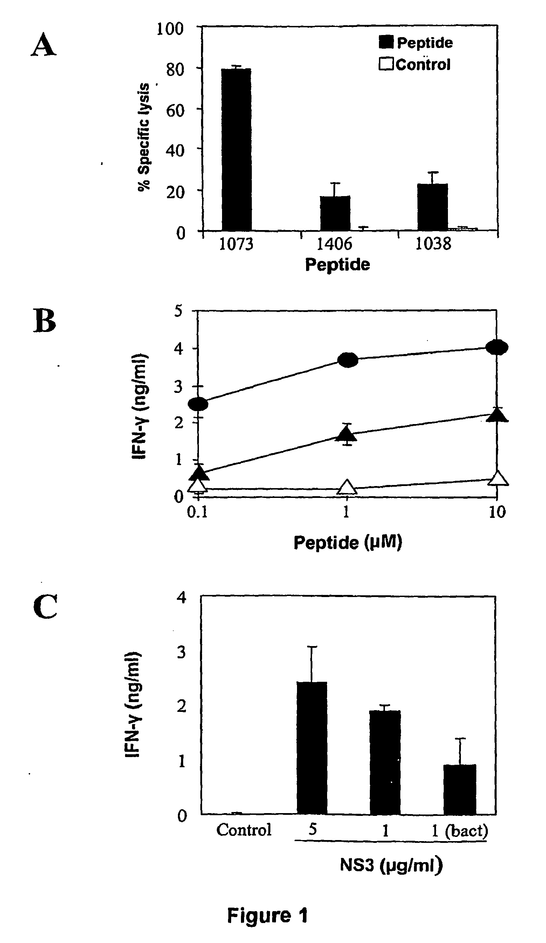

Figure 1. Immunisation with anti-CD40 and poly(I:C) together with the NS3 protein induces multi-epitopic CD4+ and CD8+ T responses. HHD mice (two per group) were injected with 50 g of anti-CD40 (i.p.). Four hours later, they were injected with 50 g of poly(I:C) (i.v.) and 500 g of recombinant NS3 protein (i.p.) (SEQ. ID. NO: 1). Six days later, the animals were killed and the splenocytes were extracted for their in vitro stimulation with different antigens and the analysis of the induced immunitary response. (A) The cells were stimulated for five days with the epitopes CD8+ 1073, 1406 or 1038 (10 $\mu$M) in the presence of IL-2. Afterwards, for each group of splenocytes, the lythic response was measured to target cells that were loaded (peptide; black bars) or not (control; white bars) with the corresponding peptide. The results obtained were shown with an effector:arget ratio of 100:1. B) In the same way, the splenocytes were cultured with different concentrations (0.1-10 $\mu$M) of the peptides 1073 (black circles), 1406 (white triangles) or 1038 (black triangles), and in the culture supernatants obtained after 48 h of stimulation the IFN-$\gamma$ content was measured by means of ELISA. (C) The splenocytes were also stimulated for 48 h with 5 or 1 $\mu$g/ml of the NS3 protein used in the immunisation (SEQ. ID. NO: 1), with 1 $\mu$g/ml of the NS3 protein produced in bacteria (SEQ. ID. NO: 3), or with culture medium (control) in order to measure the CD4+ response. Following this period of time the supernatants were collected and the amount of IFN-$\gamma$ produced was measured by means of ELISA.

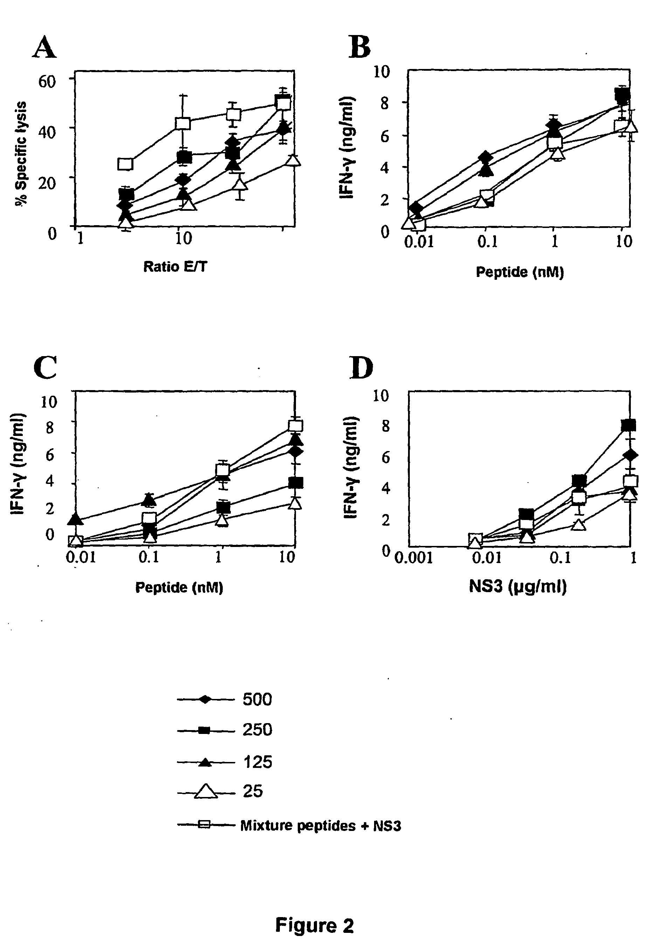

Figure 2. Measurement of the quantity of NS3 protein necessary for inducing CD4+ and CD8+ T responses in immunisation with poly(I:C) and anti-CD40. HHD mice (two per group) were immunised with NS3 protein (SEQ. ID. NO: 1) (500, 250, 125 or 25 $\mu$g/mouse) together with poly(I:C) and anti-CD40, following the protocol described in figure 1. Also included was a control group immunised in the same way, which used as antigens 5 $\mu$g of NS3 (SEQ. ID. NO: 1) and 50 $\mu$g of the peptides 1073 and 1038, along with poly(I:C) and anti-CD40. Six days later the animals were killed and the splenocytes were extracted and stimulated with different antigens (A). In order to measure the induced lythic response the cells were stimulated for five days with the epitope CD8+ 1073 (10 $\mu$M) and IL-2. Afterwards, that response was measured by confronting different quantities of effector cells against a fixed number of target cells loaded with the peptides. Moreover, the CD8+ response that had been induced was also analysed by means of the production of IFN-$\gamma$. To do this, the cells were stimulated with different concentrations of peptides 1073 (B) and 1038 (C). The cells were also stimulated with the NS3 protein (SEQ. ID. NO: 1) (D), in order to quantify the CD4+ response. After 48 h, the amount of IFN-$\gamma$ present in the supernatants was measured.

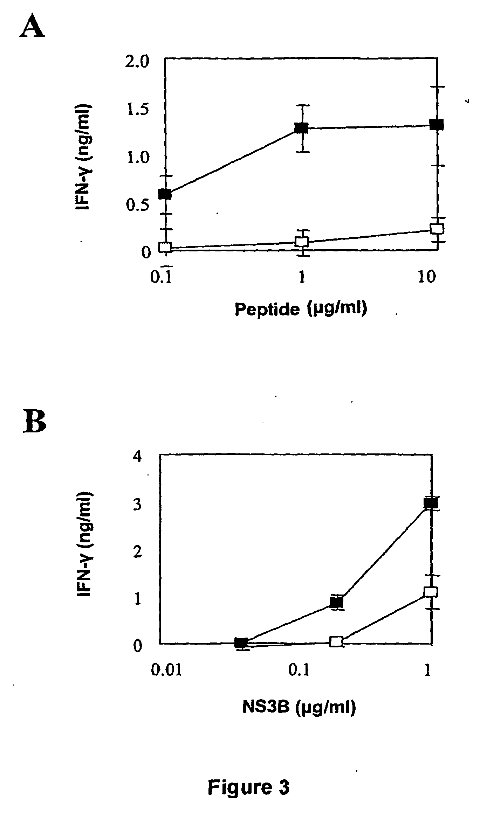

Figure 3. Immunisation with poly(I:C) and anti-CD40 together with the NS3 protein induces CD4+ and CD8+ responses in other strains of mice with different MHC. C57BL6 mice (which have MHC molecules of the type H-2b) (two per group) received one (white squares) or two (black squares) immunisations with 100 $\mu$g of NS3 (SEQ. ID. NO: 1) together with poly(I:C) and anti-CD40 following the protocol indicated in figure 1. Six days later, the animals were killed and the splenocytes were cultured with different antigens in order to measure the induced CD8+ and CD4+ responses. The restriction epitope H-2 Db 1629-1637 (GAVQNEVTL) (SEQ. ID. NO: 7) was used for stimulating the splenocytes and measuring CD8+ responses (A). The NS3 protein (SEQ. ID. NO: 1) (B) was used as stimulus for determining the CD4+ response. After two days of culture, the supernatants were collected and the amount of IFN-$\gamma$ produced was measured.

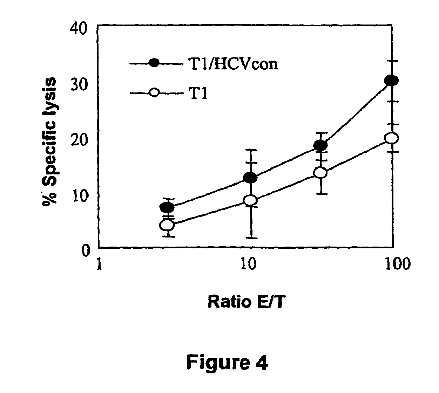

Figure 4. Immunisation with NS3 protein together with poly(I:C) and anti-CD40 induces CD8+ responses capable of recognising cells that express proteins of the HCV. (A) HHD mice (two per group) were injected with 100 $\mu$g of NS3 protein (SEQ. ID. NO: 2) plus poly(I:C) and anti-CD40 as indicated in figure 1. Six days later, the animals were killed and their splenocytes were stimulated with T1/HCVcon cells (T1 cells transfected with a plasmid that expresses the proteins of the HCV) treated with mitomycin, in the presence of IL-2. After five days of stimulation, the capacity of the splenocytes to recognise the T1/HCVcon cells was measured in lythic activity assays. To do this, different quantities of splenocytes were confronted with a fixed number of T1/HCVcon cells (black circles) or T1 control cells without being transfected (white circles).

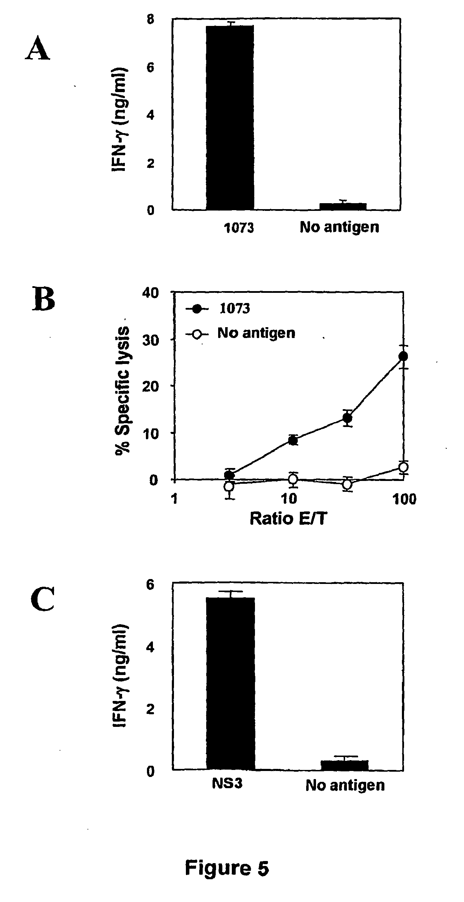

Figure 5. Immunisation with poly(I:C) and anti-CD40 together with NS3 protein induces lasting T CD4+ and CD8+ responses. HHD mice (two per group) were injected with 100 $\mu$g of NS3 protein (SEQ. ID. NO: 2) plus poly(I:C) and anti-CD40 as indicated in figure 1. Two weeks later, the animals received a second immunisation under the same conditions. Sixty days after the second immunisation the animals were killed and their splenocytes were extracted for studying the lasting CD8+ and CD4+ T response. (A) The splenocytes were stimulated with the epitope CD8+ 1073 (10 $\mu$M) or in the absence of antigen, and 48 hours later the culture supernatants were collected for measuring the amount of IFN-$\gamma$ produced. (B) The splenocytes were cultured for 5 days with the peptide 1073 (10 $\mu$M) and IL-2 and their capacity to lyse target cells loaded with the peptide 1073 was then studied. To do this, different quantities of effector cells were confronted with a fixed number of target cells loaded with the peptide 1073 (black circles) or without loading with peptide (white circles). (C) The CD4+ response was studied by means of stimulation of the splenocytes with the NS3 protein (1 $\mu$g/ml) (SEQ. ID. NO: 2) or in the absence of antigen. After 48 hours, the supernatants were collected and the amount of IFN-γ produced was measured.

## MODE OF EMBODIMENT OF THE INVENTION

The following examples, without in any way being limiting, aim to illustrate the embodiment of the invention forming the present patent application.

## RELATIVE MATERIAL AND METHODS

*Epitopes, antigens and reagents*

The peptides or epitopes used were synthesised manually in a multiple peptides synthesiser using Fmoc chemistry (Wellings DA. and Atherton E. Methods Enzymol 1997; 289: 44-67). The Kaiser ninhydrine test was used for monitoring each step. At the end of the synthesis they were spliced and deprotected with trifluoroacetic acid and washed with diethyl ether. The purity of the peptides was at all times higher than 90% determined by HPLC.

Table 1. Peptides and epitopes synthesised and used in the examples.

| Peptide or Epitope | Sequence |
| --- | --- |
| 1038-1047 | GLLGCIITSL; SEQ. ID. NO: 4 |
| 1073-1081 | CVNGVCWTV; SEQ. ID. NO: 5 |
| 1406-1415 | KLVALGINAV; SEQ. ID. NO: 6 |
| 1629-1637 | GAVQNEVTL; SEQ. ID. NO: 7 |

The numbering of the peptide or epitope refers to its relative HCVH position, taking as reference the complete sequence in the H strain of human hepatitis C which is usually taken as the prototype (GeneBank Accession Number M67463). So, for example, the database "HCV Immunology Database" (http://hcv.lanl.gov/content/ immuno/immuno-main.html) compiles the epitopes for T-Lymphocytes, both of cytotoxic T-Lymphocytes and of helper T-Lymphocytes, identified in the viral proteins of different strains and isolates of the hepatitis C virus, all of them also ordered in accordance with their relative position with respect to the H strain of the virus according to the stated GeneBank reference.

As immunogen, a recombinant polypeptide of 655 amino acids has been used which contains the complete sequence of the NS3 protein (SEQ. ID. NO: 1; Genebank accession number AAY84763.1, VRL 28-NOV-2005; 631 amino acids). As well as the 631 amino acids of the NS3 protein, the polypeptide also includes a tail with a c-myc sequence, for detection with the monoclonal antibody anti-myc, and a tail of Histidines. The protein has been produced in Pichia pastoris. It is maintained in suspension in a solution of Tris 22.5 mM / Urea 3.76 M / NaCl 300 mM. The protein has been purified by means of Ni column chromatography.

Another recombinant polypeptide has also been used as immunogen, which contains the 635 amino acids comprising the complete sequence of the NS3 protein (SEQ. ID. NO: 2; Genebank accession number D90208). As well as the amino acids corresponding to NS3, the polyprotein also includes a tail of Histidines for its purification. The DNA sequence corresponding to NS3 was obtained by digestion with Sal I and Not I of the plasmid gWIZ, which contained the NS3, sequence (supplied by Dr. G. Inchauspe, Lyon, France). The product of the digestion was cloned between the sites BsrG I and Not I of the plasmid pET-45 (+) (Novagen, Madison WI). It was expressed with E. coli and purified by means of affinity chromatography in a nickel column followed by ion exchange chromatography.

Likewise, for the in vitro assays a recombinant polypeptide (Mikrogen; Catalogue number 94302) has been used as antigen, which contains the last 20 amino acids of the non-structural protein NS2 and the first 508 amino acids of the NS3 protein of HVC (SEQ. ID. NO: 3).

As TLR3 agonist, poly(I:C) has been used obtained from Amersham (Catalogue number 27-4732-01).

As CD40 agonist, anti-CD40 antibodies were used, purified starting from the hybridome FGK-45 (Rolink A. et al., Immunity 1996. 5: 319-330).

All the reagents contained <1 unit of endotoxin per mg of product, determined by means of the lysate QCL-1000 assay of the amoebocyte limulus (Bio Whittaker).

*Mice*

C57B1/6 mice of six to eight weeks were obtained from Harlan. HHD mice were also used, transgenic for human molecules HLA-A2.1 (Pascolo S. et al., J. Exp. Med. 1997. 185: 2043-2051). All the animals were maintained under pathogen free conditions and were treated in accordance with the rules of the institution.

*Cell lines*

T2 cells were used (Salter R. et al. Immunogenetics, 1985 21: 235-246) as target cells for chromium release assays with cytotoxic T-Lymphocytes (CTL) coming from HHD mice.

T1 cells were used, transfected with a carrier plasmid of the coding region of the HCV (T1/HCVcon cells), for the recognition assays of cells which expressed the proteins of the HCV. These cells were provided by Dr. D. Moradpour (Freiburg, Germany; Volk B. et al., J Gen Virol. 2005; 86: 1737-1746). T1 cells without transfecting (ATCC, catalogue Nr. CRL-1991) were also used as control.

All the cells were grown in complete medium (RPMI 1640 10% of foetal bovine serum, 100 U/ml of penicillin, 100 $\mu$g/ml of streptomycin, 2 mM of glutamine and 50 $\mu$M of 2-mercaptoethanol). The culture of the line T1/HCVcon also contained 2 mg/ml of G418 (Gibco).

*Immunisation*

Groups of two mice were immunised via the i.p. route with 50 $\mu$g of anti-CD40. Four hours later, they were injected with 50 $\mu$g of poly(I:C) (i.v.) and different amounts of the antigens: NS3 protein or mixtures of NS3 with peptides (i.p.).

*Stimulation of splenic cells for the production of cytokines*

Splenic cells were resuspended in complete medium and plated at 8 x 105 cells/well in 0.2 ml on 96-well plates with U-shaped bottom, in the absence or presence of peptides or of the recombinant NS3 protein of the HCV.

Two days afterwards, the supernatants were collected for measuring the presence of IFN-$\gamma$ by means of ELISA (BD-Pharmingen), following the manufacturer's instructions.

*Measurement of the lythic activity of CTL*

In order to measure the CTL responses, the splenocytes coming from the immunised animals were incubated with peptides (10 $\mu$M) for 2 h at 37 °C, washed twice and cultured on 24-well plates with a confluence of 7.5 x 106 cells/well. In experiments conducted for measuring the recognition of T1/HCVcon cells, 7.5 x 106 splenocytes of HHD mice were cultured with 7.5 x 105 T1/HCVcon cells previously treated with Mitomycin C (Sigma). In all cases, two days later, 2.5 U/ml of IL-2 (Boehringer-Mannheim GmbH, Germany) was added to the wells and 5 days later the cells were recovered in order to carry out chromium release assays.

The lythic activity was measured by incubating different quantities of effector cells for 4 h with 3000 T2 target cells previously loaded with 51 Cr, with and without peptide (target). In the case of cells stimulated with T1/HCVcon, the effector cells were confronted with T1/HCVcon or T1, previously loaded with 51Cr. The culture supernatants were collected after 4 h of incubation.

The specific lysis percentage was calculated according to the formula:

$$(cpmexperimental - cpmspontaneous) / (cpmmaximum - cpmspontaneous) \times 100$$

where the spontaneous lysis (measured as cpmspontaneous) corresponds to target cells incubated in the absence of effector cells, and the maximum lysis (cpmmaximum) is obtained by incubating target cells with 5% Tritonx100.

<u>EXAMPLE 1</u>

Immunisation with anti-CD40 and poly(I:C) together with the NS3 protein induces multi-epitopic CD4+ and CD8+ T responses.

Immunisation with anti-CD40 and poly(I:C) has shown itself to be very effective for the induction of CD8+ responses by means of using as immunogens synthetic peptides which represent epitopes of CD8+ cells. Although this strategy induces potent responses, it has been demonstrated that when it is co-immunised with low quantities of NS3 protein (5 $\mu$g/mouse), which induces CD4+ response, it increases the magnitude of the CD8+ response and it also increases the high affinity CD8+ response, in other words, the one which recognises low concentrations of antigen.

Moreover, immunisation with peptides is only effective in those individuals who possess HLA molecules of the same restriction as the chosen epitopes. With the aim of tackling these two points, a study was made of whether immunisation with greater quantities of recombinant NS3 protein would be capable of inducing responses, not just CD4+ but also CD8+. To do this, mice were immunised with NS3 along with poly(I:C) and anti-CD40, and the induced responses were studied. So, HHD mice (two per group) were injected i.p. with 50 µg of anti-CD40. Four hours later, they were injected with 50 µg of poly(I:C) (i.v.) and 500 µg of recombinant NS3 protein (i.p.) (SEQ. ID. NO: 1). Six days later, the animals were killed and the splenocytes were extracted. With the aim of analysing the NS3 capacity, when the adjuvant poly(I: C) + anti-CD40 is formulated to induce CD8+ and CD4+ T responses, the splenocytes were stimulated in vitro with different antigens which specifically activates these cell populations. (A) In order to analyse the CD8+ response, in a first experiment the splenocytes were stimulated for five days with the epitopes CD8+ 1073, 1406 or 1038 in the presence of IL-2. Afterwards, for each group of cells stimulated with a peptide, their capacity was measured to lyse to target cells that were loaded with the corresponding peptide (black bars) or to control target cells without peptide (white bars). Figure 1A shows the results obtained with an effector:target ratio of 100:1. (B) The CD8+ response induced after immunisation with NS3 was also analysed by means of studying the production of IFN-γ towards the same CD8+ epitopes. To do this, the splenocytes were cultured with different quantities of 1073 (black circles), 1406 (white triangles) or 1038 (black triangles). After 48 h of culture, the supernatants were collected and the IFN-γ content was measured. (C) With the aim of analysing the induced CD4+ response, the splenocytes were stimulated with the NS3 protein used in the immunisation (SEQ. ID. NO: 1). Also, the cells were stimulated with commercial NS3 protein produced in bacteria (SEQ. ID. NO: 3). In the same way as in the previous point, the degree of activation was measured by means of the production of IFN-γ.

First of all, it was possible to check that this antigen was capable of inducing CD8+ responses, which could be detected both in chromium release assays (Figure 1A) and by means of the induction of the production of IFN-γ (Figure 1B). Moreover, this response was multi-epitopic, being directed towards various CD8+ epitopes, which have been characterised within the NS3 sequence (e.g.: peptides 1073, 1406 and 1038). Finally, it was also confirmed that it was capable of inducing CD4+ responses, which recognised the NS3 protein used in the immunisation and the commercial NS3 protein produced in bacteria (Figure 1 C). The response towards this latter was lower, presumably due to the fact that there existed some changes in the sequence of both proteins and that the protein expressed in bacteria was shorter, with which it could lose some epitopes recognised by the CD4+ T-Lymphocytes.

EXAMPLE 2

The administration of 25 µg of recombinant NS3 together with poly(I:C) and anti-CD40 is sufficient for inducing CD4+ and CD8+ T responses.

From previous experiments we knew that with 5 µg of NS3 CD4+ responses were induced but not CD8+, and we therefore wished to discover the minimum quantity of NS3 that would be sufficient for inducing CD8+ responses. To do this, HHD mice were immunised with 500, 250, 125 and 25 µg of NS3 (SEQ. ID. NO: 1). Also included as control was a group immunised with peptides corresponding to CD8+ epitopes, which would induce CD8+ responses, plus 5 µg of NS3 (SEQ. ID. NO: 1), which would induce CD4+ responses. For this, in each group of animals immunised with a dose of NS3 an analysis was conducted of the CD8+ response and the CD4+ response. The CD8+ response was analysed as the capacity to lyse to target cells loaded with the epitope CD8+ 1073 (Fig. 2A), along with the capacity to produce IFN-γ with regard to different concentrations of the epitopes CD8+ 1073 (Fig 2B) and 1038 (Fig 2C). The CD4+ responses were measured by means of the capacity to produce IFN-γ with regard to different concentrations of NS3 (SEQ. ID. NO: 1) (Fig 2D). This experiment demonstrated that all the quantities of NS3 assayed were capable of inducing CD8+ responses, when the lythic responses to the peptide 1073 were studied (Fig 2A), the dose of 25 µg being the one that induced responses of the weakest intensity. Moreover, all the doses were capable of inducing the production of IFN-γ with regard to the epitopes 1073 (Fig 2B) and 1038 (Fig 2C), which indicated that the capacity to induce multi-epitopic responses was maintained even when the doses were reduced. Finally, and as was expected, all of them induced CD4+ responses. Given that, in the majority of cases, the induced response was less when 25 µg of NS3 was used, for later experiments a dose of 100 µg/mouse was chosen, starting from which dose no increase was observed in the induction of responses.

EXAMPLE 3

Immunisation with poly(I:C) and anti-CD40 together with the NS3 protein induces CD4+ and CD8+ responses in other strains of mice with different MHC.

Given that in an antigen as large as the NS3 protein, it is possible to find CD4+ and CD8+ epitopes, which can be presented by different molecules of MHC, the capacity of this immunisation protocol for inducing CD4+ and CD8+ responses in another strain of mouse with different MHC molecules was studied. To do this, C57/BI6 mice, which have H-2b restriction MHC molecules, were immunised with 100 μ9 of NS3 (SEQ. ID. NO: 1). With the aim of improving the responses, one group received a single immunisation and the other group received a second booster immunisation. First of all, the CD8+ response was measured, as the production of IFN-γ against the peptide 1629-1637 (SEQ. ID. NO: 7), which contains a CD8+ epitope presented by the MHC molecules of class I H-2 Db. As can be seen in Figure 3A, a detectable response was induced in both groups of mice, though the levels were considerably greater in the group that had received two immunisations (black squares) than in the one that received one immunisation (white squares). The CD4+ response, measured as the production of IFN-γ against the recombinant NS3 protein (SEQ. ID. NO: 1) was also detected in the two groups (Figure 3B), and again demonstrated that two immunisations (black squares) induced more potent responses that a single immunisation (white squares).

EXAMPLE 4

Immunisation with NS3 protein together with poly(I:C) and anti-CD40 induces CD8+ responses capable of recognising cells that express proteins of the HCV.

With the aim of studying whether immunisation using NS3 protein together with poly(I:C) and anti-CD40 would be capable of inducing responses that could potentially kill cells infected with HCV, an in vitro model was used of target cells transfected with a plasmid that expressed the proteins of the HCV (T1/HCVcon). These cells expressed the same peptides in their Class I MHC molecules as would be expressed by a cell infected with HCV; therefore, it could be assumed as a response against the latter any certain response against them. The NS3 protein (SEQ. ID. NO: 1) used in the experiments of figures 1 to 3 corresponds to a different viral strain from the viral strain present in the T1/HCVcon cells. These two strains present some differences in the CD8+ epitopes studied so far. With the aim of optimising the recognition capacity of the CD8+ epitopes present in the T1/HCVcon cells, for this experiment an NS3 protein (SEQ. ID. NO: 2) was used as immunogen, whose sequence had a degree of homology greater than the protein present in the T1/HCVcon cells. Six days after immunisation of HHD mice with 100 μg of NS3, the splenocytes were stimulated with T1/HCVcon cells. The recognition capacity of T1/HCVcon cells was analysed in lythic activity assays. To do this, stimulated splenocytes were confronted with T1/HCVcon cells and T1 control cells. As shown in figure 4, immunisation with NS3 induced responses with a greater capacity to lyse T1 cells, which expressed proteins of the HCV (black circles) than T1 control cells (white circles).

EXAMPLE 5

Immunisation with poly(I:C) and anti-CD40 together with NS3 protein induces lasting T CD4+ and CD8+ responses.

One of the main properties that a vaccination protocol has to possess is its capacity to induce lasting immunitary responses, so that the protection conferred by the immunisation can persist in the long term. In order to study whether immunisation with anti-CD40 and poly(I:C) together with the NS3 protein would be capable of inducing this kind of response, HHD mice were immunised with 100 μg of NS3 in accordance with the protocol described in example 1. With the aim of reinforcing the response, after 15 days the animals received a booster dose under the same conditions. Sixty days after the second immunisation the animals were killed and their splenocytes were stimulated with different antigens in order to analyse the CD8+ and CD4+ T responses persisting at that moment. In order to study the CD8+ T response, the cells were stimulated with the epitope 1073 and the production of IFN-γ and the lythic activity were measured. As shown in figure 5A, sixty days after the second immunisation, the splenocytes of mice immunised with anti-CD40 and poly(I:C) together with the NS3 protein were capable of producing large amounts of IFN-γ when stimulated with the peptide 1073, but not in the absence of antigen. Moreover, these cells were capable of lysing target cells pulsed with the peptide 1073 (black circles) but not target cells that did not contain antigen (white circles) (Figure 5B). Finally, the CD4+ response was also studied, using as antigen the NS3 protein used in the immunisation. Figure 5C shows that this immunisation protocol also induces potent and lasting CD4+ responses, which specifically recognise NS3.

**Claims**

1. An immuno-stimulant combination for prophylaxis and treatment of hepatitis C, **characterised in that** it comprises:

    a) Poly(I:C) acting as a TLR3 agonist,
    b) a CD40 agonist or a sequence of DNA that codes it, and
    c) a polypeptide, which comprises the NS3 protein of the hepatitis C virus, or a fragment of said NS3 protein with capacity for inducing CD8+ and CD4+ responses.

**2.** An immuno-stimulant combination according to claim 1, **characterised in that** the CD40 agonist is selected from between an anti-CD40 antibody, CD40L, and fragments of the above which conserve their capacity for joining to CD40.

**3.** An immuno-stimulant combination according to claim 2, **characterised in that** the CD40 agonist is an anti-CD40 antibody.

**4.** An immuno-stimulant combination according to claim 1, **characterised in that** it comprises:

> a) poly(I:C),
> b) an anti-CD40 antibody, and
> c) a polypeptide containing the NS3 protein.

**5.** An immuno-stimulant combination according to claim 3 or 4, **characterised in that** the polypeptide containing the NS3 protein is a polypeptide with SEQ ID. NO: 1.

**6.** An immuno-stimulant combination according to any of claims 1 to 5 **characterised in that** all the components form part of a single pharmaceutical composition.

**7.** An immuno-stimulant combination according to any of claims 1 to 5 **characterised in that** all the components form part of at least two different pharmaceutical compositions.

**8.** Use of an immuno-stimulant combination defined in any of claims 1 to 7 in the preparation of a medicine for the prophylaxis and treatment of hepatitis C.

**9.** Use of an immuno-stimulant combination defined in any of claims 1-7 in the preparation of a medicine for the treatment of hepatitis C.

**10.** Use of an immuno-stimulant combination according to claims 8 or 9, **characterised in that** said medicine comprises at least two pharmaceutical compositions suitable for simultaneous administration.

**11.** Use of an immuno-stimulant combination according to claims 8 or 9, **characterised in that** said medicine comprises at least two pharmaceutical compositions suitable for separate administration.

**12.** Use of an immuno-stimulant combination according to claim 11, **characterised in that** said pharmaceutical compositions are suitable for separate administration by different routes.

**13.** A pharmaceutical composition containing an immuno-stimulant combination described in any of claims 1 to 5, **characterised in that** each one of the components is present in pharmaceutically acceptable quantities.

**14.** A kit for the administration of an immuno-stimulant combination described in any of claims 1 to 5, **characterised in that** it comprises at least two different pharmaceutical compositions as defined in claim 13, each one containing at least one of the components a), b) or c) of said immuno-stimulant combination as defined in claim 1.

**15.** An immuno-stimulant combination defined in any of claims 1 to 7, in an effective quantity for inducing an immune response to the hepatitis C virus.

**16.** An immuno-stimulant combination according to claim 15, for use as a prophylactic treatment.

**17.** An immuno-stimulant combination according to claim 15, for use as a therapeutic treatment.

**18.** A vaccine against hepatitis C virus, **characterised in that** it comprises an immuno-stimulant combination defined in any of claims 1 to 7.

**Patentansprüche**

**1.** Immunstimulierende Kombination zur Prophylaxe und Behandlung von Hepatitis C, **dadurch gekennzeichnet,**

**dass** sie umfasst:

a) Poly(I:C), welches als TLR3-Agonist wirkt,
b) einen CD40-Agonisten oder eine Sequenz von DNA, die für diesen kodiert, und
c) ein Polypeptid, welches das NS3-Protein des Hepatitis-C-Virus oder ein Fragment von besagtem NS3-Protein umfasst, mit der Fähigkeit, CD8+ und CD4+ Antworten herbeizuführen.

2. Immunstimulierende Kombination gemäß Anspruch 1, **dadurch gekennzeichnet, dass** der CD40-Agonist ausgewählt ist aus zwischen einem Anti-CD40-Antikörper, CD40L und Fragmenten der obigen, welche ihre Fähigkeit bewahren, an CD40 zu binden.

3. Immunstimulierende Kombination gemäß Anspruch 2, **dadurch gekennzeichnet, dass** der CD40-Agonist ein Anti-CD40-Antikörper ist.

4. Immunstimulierende Kombination gemäß Anspruch 1, **dadurch gekennzeichnet, dass** sie umfasst:

a) Poly(I:C),
b) einen Anti-CD40-Antikörper und
c) ein Polypeptid, welches das NS3-Protein enthält.

5. Immunstimulierende Kombination gemäß Anspruch 3 oder 4, **dadurch gekennzeichnet, dass** das Polypeptid, welches das NS3-Protein enthält, ein Polypeptid mit SEQ ID Nr. 1 ist.

6. Immunstimulierende Kombination gemäß einem von Ansprüchen 1 bis 5, **dadurch gekennzeichnet, dass** alle Bestandteile Teil einer einzelnen pharmazeutischen Zusammensetzung bilden.

7. Immunstimulierende Kombination gemäß einem von Ansprüchen 1 bis 5, **dadurch gekennzeichnet, dass** alle Bestandteile Teil von mindestens zwei verschiedenen pharmazeutischen Zusammensetzungen bilden.

8. Verwendung einer immunstimulierenden Kombination, definiert in einem von Ansprüchen 1 bis 7, in der Herstellung einer Medizin zur Prophylaxe und Behandlung von Hepatitis C.

9. Verwendung einer immunstimulierenden Kombination, definiert in einem von Ansprüchen 1-7, in der Herstellung einer Medizin zur Behandlung von Hepatitis C.

10. Verwendung einer immunstimulierenden Kombination gemäß Ansprüchen 8 oder 9, **dadurch gekennzeichnet, dass** besagte Medizin mindestens zwei pharmazeutische Zusammensetzungen umfasst, welche für gleichzeitige Verabreichung geeignet sind.

11. Verwendung einer immunstimulierenden Kombination gemäß Ansprüchen 8 oder 9, **dadurch gekennzeichnet, dass** besagte Medizin mindestens zwei pharmazeutische Zusammensetzungen umfasst, welche für getrennte Verabreichung geeignet sind.

12. Verwendung einer immunstimulierenden Kombination gemäß Anspruch 11, **dadurch gekennzeichnet, dass** besagte pharmazeutische Zusammensetzungen für getrennte Verabreichung auf verschiedenen Wegen geeignet sind.

13. Pharmazeutische Zusammensetzung, welche eine in einem von Ansprüchen 1 bis 5 beschriebene immunstimulierende Kombination enthält, **dadurch gekennzeichnet, dass** jeder der Bestandteile in pharmazeutisch annehmbaren Mengen vorhanden ist.

14. Kit zur Verabreichung einer in einem von Ansprüchen 1 bis 5 beschriebenen immunstimulierenden Kombination, **dadurch gekennzeichnet, dass** es mindestens zwei verschiedene pharmazeutische Zusammensetzungen wie in Anspruch 13 definiert umfasst, wovon jede mindestens einen der Bestandteile a), b) oder c) von besagter immunstimulierender Kombination wie in Anspruch 1 definiert enthält.

15. Immunstimulierende Kombination, definiert in einem von Ansprüchen 1 bis 7, in einer wirksamen Menge zum Herbeiführen einer Immunantwort auf das Hepatitis-C-Virus.

**16.** Immunstimulierende Kombination gemäß Anspruch 15 zur Verwendung als prophylaktische Behandlung.

**17.** Immunstimulierende Kombination gemäß Anspruch 15 zur Verwendung als therapeutische Behandlung.

**18.** Vakzin gegen Hepatitis-C-Virus, **dadurch gekennzeichnet, dass** es eine in einem von Ansprüchen 1 bis 7 definierte immunstimulierende Kombination umfasst.

**Revendications**

**1.** Combinaison immunostimulante pour la prophylaxie et le traitement de l'hépatite C, **caractérisée en ce qu'**elle comprend :

a) un poly(I:C) agissant comme un agoniste de TLR3,
b) un agoniste de CD40 ou une séquence d'ADN qui le code, et
c) un polypeptide, qui comprend la protéine NS3 du virus de l'hépatite C, ou un fragment de ladite protéine NS3 avec la capacité d'induire les réponses des CD8+ et CD4+.

**2.** Combinaison immunostimulante selon la revendication 1, **caractérisée en ce que** l'agoniste de CD40 est choisi parmi un anticorps anti-CD40, le CD40L, et des fragments de ceux-ci qui conservent leur capacité à se joindre au CD40.

**3.** Combinaison immunostimulante selon la revendication 2, **caractérisée en ce que** l'agoniste de CD40 est un anticorps anti-CD40.

**4.** Combinaison immunostimulante selon la revendication 1, **caractérisée en ce qu'**elle comprend :

a) un poly(I:C),
b) un anticorps anti-CD40, et
c) un polypeptide contenant la protéine NS3.

**5.** Combinaison immunostimulante selon les revendications 3 ou 4, **caractérisée en ce que** le polypeptide contenant la protéine NS3 est un polypeptide avec la SEQ ID NO:1.

**6.** Combinaison immunostimulante selon l'une quelconque des revendications 1 à 5 **caractérisée en ce que** tous les composants forment une partie d'une seule composition pharmaceutique.

**7.** Combinaison immunostimulante selon l'une quelconque des revendications 1 à 5 **caractérisée en ce que** tous les composants forment une partie d'au moins deux compositions pharmaceutiques différentes.

**8.** Utilisation d'une combinaison immunostimulante définie dans l'une quelconque des revendications 1 à 7 dans la préparation d'un médicament pour la prophylaxie et le traitement de l'hépatite C.

**9.** Utilisation d'une combinaison immunostimulante définie dans l'une quelconque des revendications 1 à 7 dans la préparation d'un médicament pour le traitement de l'hépatite C.

**10.** Utilisation d'une combinaison immunostimulante selon l'une quelconque des revendications 8 ou 9, **caractérisée en ce que** ledit médicament comprend au moins deux compositions pharmaceutiques appropriées pour l'administration simultanée.

**11.** Utilisation d'une combinaison immunostimulante selon l'une quelconque des revendications 8 ou 9, **caractérisée en ce que** ledit médicament comprend au moins deux compositions pharmaceutiques appropriées pour l'administration séparée.

**12.** Utilisation d'une combinaison immunostimulante selon la revendication 11, **caractérisée en ce que** lesdites compositions pharmaceutiques sont appropriées pour l'administration séparée par différentes voies.

**13.** Composition pharmaceutique contenant une combinaison immunostimulante décrite dans l'une quelconque des revendications 1 à 5, **caractérisée en ce que** chacun des composants est présent dans des quantités pharmaceutiquement acceptables.

14. Trousse pour l'administration d'une combinaison immunostimulante décrite dans l'une quelconque des revendications 1 à 5, **caractérisée en ce qu'**elle comprend au moins deux compositions pharmaceutiques différentes comme définies dans la revendication 13, chacune comprenant au moins un des composants a), b) ou c) de ladite combinaison immunostimulante comme définie dans la revendication 1.

15. Combinaison immunostimulante définie dans l'une quelconque des revendications 1 à 7, dans une quantité efficace pour induire une réponse immunitaire au virus de l'hépatite C.

16. Combinaison immunostimulante selon la revendication 15, pour l'utilisation comme traitement prophylactique.

17. Combinaison immunostimulante selon la revendication 15, pour l'utilisation comme traitement thérapeutique.

18. Vaccin contre le virus de l'hépatite C, **caractérisé en ce qu'**il comprend une combinaison immunostimulante définie dans l'une quelconque des revendications 1 à 7.

**Figure 1**

Figure 2

**A**

**B**

**Figure 3**

Figure 4

**Figure 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2002014362 A2

- US 20040141950 A

**Non-patent literature cited in the description**

- Insights: Hepatitis C. *Nature,* 2005, vol. 436 (7053), 929-978
- **Grakoui A. et al.** HCV persistence and immune evasion in the absence of memory T-cell help. *Science,* 2003, vol. 302, 659-662
- **Rouas et al.** *International Immunology,* May 2004, vol. 16 (5), 767-773
- **Ahonen et al.** *J. Exp. Med.,* 2004, vol. 199, 775-784
- **Alexopoulou et al.** *Nature,* 2001, vol. 413, 732-738

- **Wellings DA. ; Atherton E.** *Methods Enzymol,* 1997, vol. 289, 44-67
- **Rolink A. et al.** *Immunity,* 1996, vol. 5, 319-330
- **Pascolo S. et al.** *J. Exp. Med.,* 1997, vol. 185, 2043-2051
- **Salter R. et al.** *Immunogenetics,* 1985, vol. 21, 235-246
- **Freiburg, Germany ; Volk B. et al.** *J Gen Virol.,* 2005, vol. 86, 1737-1746